Europäisches Patentamt

European Patent Office

Office européen des brevets

(11) Publication number: **0 386 662**
A1

## (12) EUROPEAN PATENT APPLICATION

(21) Application number: 90104143.4

(51) Int. Cl.5: **B65D 5/10, A61B 19/02**

(22) Date of filing: 03.03.90

(30) Priority: 08.03.89 IT 1968589

(43) Date of publication of application:
**12.09.90 Bulletin 90/37**

(84) Designated Contracting States:
**BE CH DE ES FR GB GR LI NL**

(71) Applicant: **ASSOGRAPH ITALIA S.R.L.**
**Via Biancamano, 14**
**I-20052 Monza Milano(IT)**

(72) Inventor: **Goretti, Franco**
**Via Don Gnocchi 23**
**I-20045 Besana Brianza (Milano)(IT)**

(74) Representative: **Modiano, Guido et al**
**MODIANO, JOSIF, PISANTY & STAUB**
**Modiano & Associati Via Meravigli, 16**
**I-20123 Milano(IT)**

(54) Container for special hospital waste.

(57) A container for special hospital waste which has constructive solutions suitable to ensure its snap-together safety closure, as regards both the lid and the bottom. The container is constituted by a sheet (1) of a suitable material which is appropriately punched and creased and defines four intermediate portions (5). These portions allow to provide a tubular body with a rectangular plan, of appropriate cross section and height, which is provided, above and below, with co-operating perimetral flaps (7,7',10,11,16,16'18,19). In particular, these flaps can be coupled, in opposite pairs, so as to form a bottom and a lid with irreversible closure.

Fig. 1

EP 0 386 662 A1

## CONTAINER FOR SPECIAL HOSPITAL WASTE

The present invention relates to a container for special hospital waste, and in particular to a container having a snap-together safety closure.

As known, there are refuse products, or waste, in particular hospital waste, for the disposal of which it is necessary to resort to packaging containers which can be sealed with absolute safety to prevent any possibility of reopening them either involuntarily or accidentally.

This need obviously entails a considerable waste of time and requires the availability of adhesive tapes, straps, staplers, glues and the like.

Conventional containers are furthermore usually marketed in assembled form, so that they are extremely bulky during storage.

The aim of the present invention is to eliminate the above described disadvantages by providing a container for special hospital waste the closure whereof, after filling, is securely irreversible.

Within the scope of this aim, a particular object of the present invention is to provide a container for special hospital waste which can be reopened exclusively by breaking and/or removing parts.

Another object of the present invention is to provide a container for special hospital waste which is easy to assemble and can therefore be stored in the form of a sheet having a flattened shape.

This aim, as well as the objects mentioned and others which may become apparent hereinafter, are achieved by a container for special hospital waste, according to the invention, characterized in that it is constituted by a sheet of an appropriate material, conveniently punched and creased, on which four intermediate portions are defined, said portions being adapted to allow the forming of a tubular body with rectangular plan and with appropriate cross section and height, provided, above and below, with perimetral co-operating flaps; said flaps, in particular, can be coupled in opposite pairs so as to form a bottom and a lid with unopenable closure.

Further characteristics and advantages of the self-sealing waste container, which constitutes the subject of the present invention, will become apparent with the aid of the following description of a preferred embodiment of said container, illustrated only by way of non-limitative example in the various figures of the accompanying drawings, wherein:

figure 1 is a view of the flattened shape of the punched and creased sheet material for forming the container;

figure 2 is a perspective view of said container after closing the lid;

figures 3, 3', 4 and 5 are views of the various steps of assembly of the flaps which form the bottom of the container;

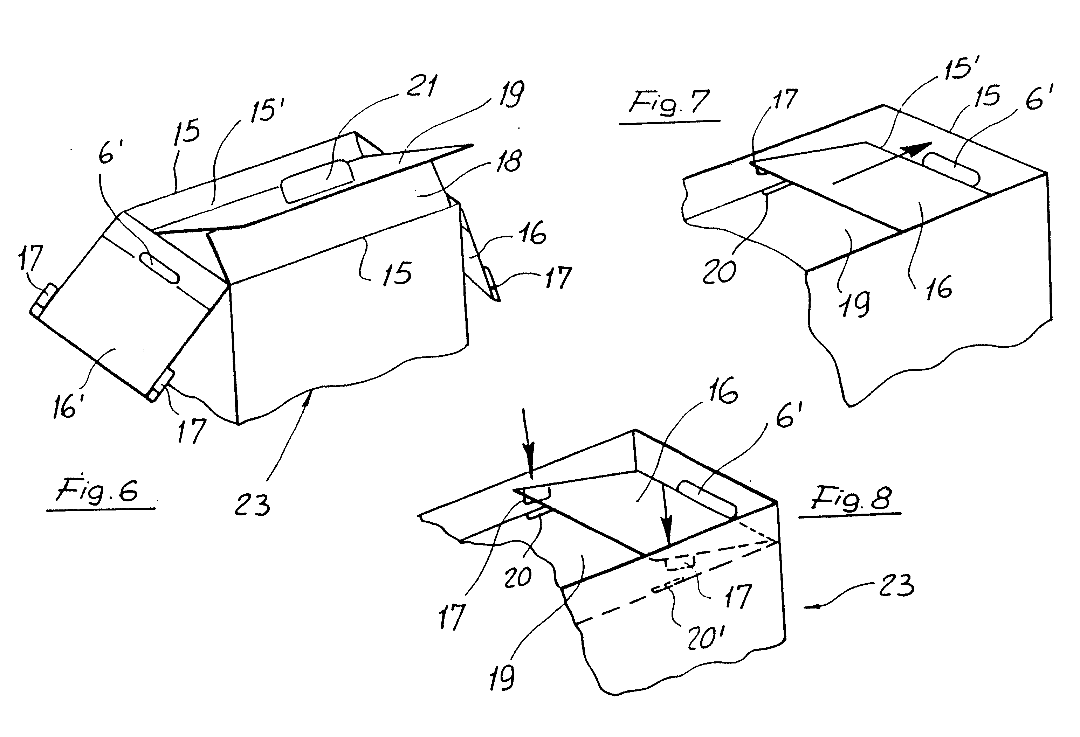

figures 6, 7 and 8 are views of the steps of assembly of the lid.

With particular reference to the numeric symbols of the various figures of the accompanying drawings, the container for special hospital waste according to the invention is obtained starting from a conveniently punched sheet 1 of an appropriate material (cardboard, card, or the like) possibly treated on one or both sides, according to the specific requirements.

More precisely, said sheet is punched so as to define an intermediate rectangular band which is provided, above and below, with four flaps arranged side by side.

Said intermediate rectangular band has, at an end, in a longitudinal direction, a protruding flap 2 treated with adhesive, which can stably couple to the opposite end 3 of said band, the folding whereof is determined by the creases 4.

It should be pointed out that a corresponding number of slots 6 is provided in an upward position on the two portions 5 of the band delimited by alternated creases.

Those of said lower flaps or folds which correspond to said portions 5, 7 and 7', respectively have, in correlated positions, a slot 8 and a tab 9.

The other two lower flaps 10 and 11 have, in turn, a slot 12 and a tab 13 which defines a tongue-like configuration.

Said lower flaps, in particular, fold about a crease 14, whereas the upper flaps can be folded twice about the creases 15 and 15'.

Those upper flaps which are connected to the portions 5 of said central band, which are indicated by 16 and 16', have, between the two creases, corresponding slots 6' and are laterally provided with protruding tongues 17.

The other two upper flaps 18 and 19 have, in turn, pairs of slots 20 and 20' which are provided proximate to the crease 15' and parallel thereto, and respectively define a tab 21 and a slot 22.

Essentially, by folding the sheet along the creases 4 and by fixing the flap 2 onto the edge 3, a tubular body, generally indicated by the reference numeral 23, is initially obtained.

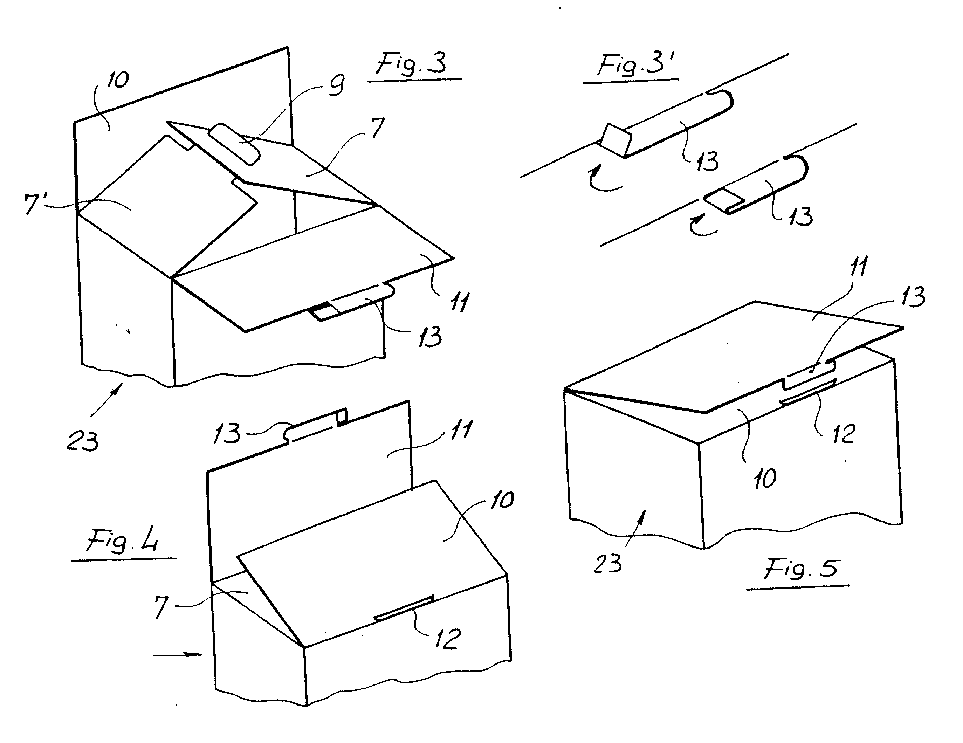

Then the bottom is formed (figures 3 to 5) by folding the flaps 7 and 7', inserting the tab 9 in the slot 8, followed by the folding of the flap 10 on said flaps and by the folding of the flap 11 on said flap 10.

In particular, one end 13' of the tab 13 is folded on itself so as to allow the insertion of said tab into the slot 12.

After said insertion, said end is naturally returned to an extended position, locking the flap 11 on the underlying flaps, so as to prevent every possibility of reopening of the lid except by breaking its component parts.

After filling the container, the flaps 18 and 19 are folded, inserting the tab 21 in the slot 22, and said flaps are inserted between the walls of the box-like body, folding them about the crease 15'.

In practice, by means of this solution, a convenient stiffening of the walls of the box-like body is achieved and a depressed seat is created in which the flaps 16 and 16' also insert with a double fold.

The tabs 17 of these last, in particular, after folding, are inserted in the corresponding slots 20 and 20'.

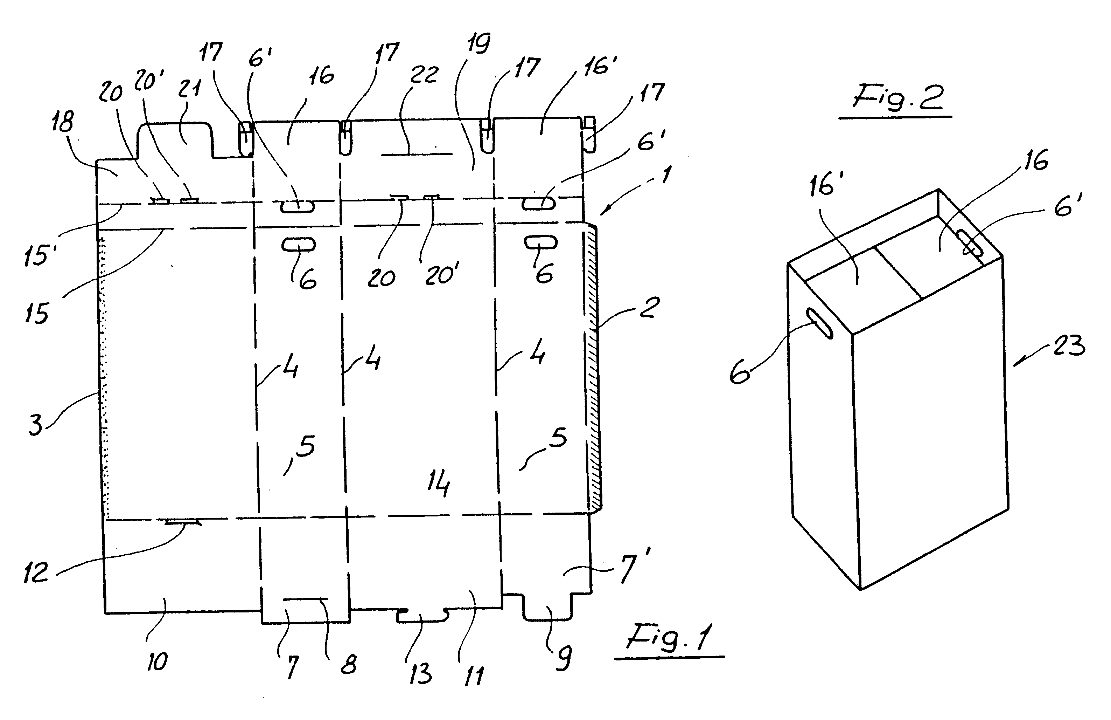

Then, by pushing said flaps 16 and 16' against the walls of the box like body (figure 8), the contiguous edges of said flaps are arranged adjacent on a plane (figure 2) and the tabs 17 are simultaneously engaged against the inner face of the flaps 18 and 19.

A closure of a substantially self locking type, capable of preventing subsequent reopening of the lid, is consequently provided, and the container thus obtained is given considerable structural strength.

From what has been described above and from the observation of the various figures of the accompanying drawings, the great functionality and practicality of use which characterize the container for special hospital waste which constitutes the subject of the present invention are evident.

Said self sealing container has been described above and illustrated merely by way of non-limitative example and with the exclusive purpose of demonstrating the possibility of practical execution and the general characteristics of the present invention; therefore all those variations and modifications which are within the scope of an expert in the field and are susceptible to be within the scope of the above described innovative concepts may be carried out on said container.

Where technical features mentioned in any claim are followed by reference signs, those reference signs have been included for the sole purpose of increasing the intelligibility of the claims and accordingly such reference signs do not have any limiting effect on the scope of each element identified by way of example by such reference signs.

portions are defined, said portions being adapted to provide a tubular body with rectangular plan and with appropriate cross section and height which is provided, above and below, with perimetral cooperating flaps; said flaps, in particular, can be coupled in opposite pairs so as to form a bottom and a lid with irreversible closure.

2. Container for special hospital waste, according to claim 1, characterized in that said sheet is made of cardboard, card or the like, possibly treated on one or both surfaces, and punched so as to define an intermediate rectangular band which is provided, above and below, with four flaps arranged side by side; said intermediate rectangular band has, at one end, in a longitudinal direction, a protruding flap treated with adhesive, which can be stably coupled to the opposite end of said band, the folding whereof is determined by appropriate creases.

3. Container for special hospital waste, according to the preceding claims, characterized in that a corresponding number of slots is provided in an upward position on two portions of said band delimited by alternated creases.

4. Container for special hospital waste, according to one or more of the preceding claims, characterized in that those of said lower flaps which correspond to said portions of the intermediate band respectively have, in correlated positions, a slot and a tab; the other two lower flaps have, in turn, a slot and a tab which is similar to a tongue.

5. Container for special hospital waste, according to one or more of the preceding claims, characterized in that said lower flaps fold about a single crease, whereas the upper flaps can be folded twice about the same number of creases.

6. Container for special hospital waste, according to one or more of the preceding claims, characterized in that those upper flaps which are related to said portions of said central band have corresponding slots between the two creases and are laterally provided with protruding tabs; the other two upper flaps have, in turn, pairs of slots provided proximate to the inner crease and parallel thereto and respectively define, in correlated positions, a tab and a slot.

## Claims

1. Container for special hospital waste, characterized in that it is constituted by a sheet made of a suitable material which is appropriately punched and creased, on which four intermediate

Fig.2

Fig.1

Fig. 3

Fig. 3'

Fig. 4

Fig. 5

EP 0 386 662 A1

Fig.6

Fig.7

Fig.8

EP 0 386 662 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | FR-A-1 043 451 (IMPRIMERIE-CARTONNERIE TOULOISE CARTOLA) * Figures 1,5; page 1, right-hand column, line 25 - page 2, left-hand column, line 20 * --- | 1,2 | B 65 D 5/10 A 61 B 19/02 |
| A | DE-A-2 903 562 (HAMMERLIT GmbH) * Figures 2,8,12,13; Page 6, lines 1-3; page 12, lines 10-19; page 13, lines 1-8 * --- | 1-3 | |
| X | GB-A-2 085 409 (PENCORY LTD) * Figure 1; page 1, line 88 - page 2, line 7 * | 1,2 | |
| A | --- | 5 | |
| X | FR-A-2 623 479 (ROCHETTE CENPA ONDULE) * Figure 1; page 2, line 24 - page 4, line 2 * ----- | 1,2 | |

TECHNICAL FIELDS
SEARCHED (Int. Cl.5)

B 65 D
A 61 B
B 65 F

The present search report has been drawn up for all claims

| Place of search | Date of completion of the search | Examiner |
|---|---|---|
| THE HAGUE | 31-05-1990 | PERNICE,C. |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons
........................................................................
& : member of the same patent family, corresponding
  document

EPO FORM 1503 03.82 (P0401)